# EUROPÄISCHE PATENTANMELDUNG

(11) **EP 1 428 814 A1**
(43) Veröffentlichungstag der Anmeldung: **16.06.2004**
(21) Anmeldenummer: 03026982.3
(22) Anmeldetag: 26.11.2003
(51) Int. Cl.: C07C 47/565, C07C 45/56, C07C 47/575, C07C 39/27, C07C 37/50, C07C 213/02, C07C 215/50, C07C 217/58

(54) **Fluorhaltige Benzaldehyde**

(30) Priorität: 09.12.2002 DE 10257357
(71) Anmelder: Bayer Chemicals AG, 51368 Leverkusen (DE)
(72) Erfinder: Peilstöcker, Karen, Dr., 51061 Köln (DE); Marhold, Albrecht, Dr., 51373 Leverkusen (DE)

(57) **Zusammenfassung**

Die vorliegende Erfindung betrifft fluorhaltige Benzaldehyde, ein Verfahren zu deren Herstellung sowie die Anwendung der fluorhaltigen Benzaldehyde zur Herstellung von Wirkstoffen insbesondere in Arzneimitteln und Agrochemikalien.

## Beschreibung

Die vorliegende Erfindung betrifft fluorhaltige Benzaldehyde, ein Verfahren zu deren Herstellung sowie die Anwendung der fluorhaltigen Benzaldehyde zur Herstellung von Wirkstoffen insbesondere in Arzneimitteln und Agrochemikalien.

Fluorhaltige Benzaldehyde, wie zum Beispiel fluorhaltige 2-Hydroxy- oder 2-Alkoxy-benzaldehyde sind wertvolle Ausgangsprodukte für die Herstellung von Wirkstoffen in Arzneimitteln und Agrochemikalien, da die Fluor- oder fluorhaltigen Substituenten die Lipophilie und damit die Membrangängigkeit des gesamten Wirkstoffinoleküls erhöhen. So eignen sich solche Verbindungen wie zum Beispiel 5-Fluor-2-hydroxy-benzaldehyd beispielsweise als Ausgangsprodukt zur Herstellung von Arzneimitteln, die zur Behandlung von Herz-Kreislauf-Erkrankungen eingesetzt werden (siehe auch WO-A 01/19780, S. 81).

Die Herstellung von 5-Fluor-2-hydroxybenzaldehyd kann beispielsweise durch Formylierung von 4-Fluor-phenol erfolgen (Suzuki et al., Chem. Pharm. Bull. 1963, 31(5), 1751-1753). Die Ausbeuten sind mit weniger als 20 % d. Th. jedoch nicht akzeptabel.

Es bestand daher das Bedürfnis, fluorhaltige 2-Hydroxy-3-methylbenzaldehyde und ein effizientes Verfahren zu deren Herstellung bereitzustellen.

Es wurde nun ein Verfahren zur Herstellung von Verbindungen der Formel (I) gefunden, 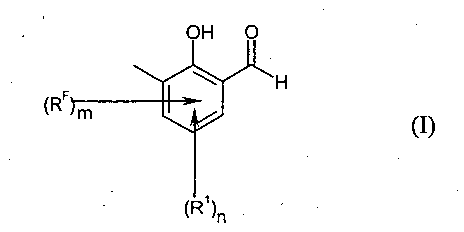 in der
- R¹: jeweils unabhängig für C₁-C₁₂-Alkyl, Chlor oder Brom oder für Reste der Formeln (IIa) oder (IIb) steht,

A-B-D-E (IIa)

A-E (IIb)

in denen unabhängig voneinander
A fehlt oder für einen C₁-C₈-Alkylenrest steht und
B fehlt oder für Sauerstoff, Schwefel oder NR² steht, wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
- n: für eine ganze Zahl von 0 bis 3-m steht und
- R^{F}: für Fluor, C₁-C₁₂-Fluoralkyl, -O(C₁-C₁₂-Fluoralkyl) oder -S(C₁-C₁₂-Fluoralkyl) steht und
- m: für eine ganze Zahl von 1 bis 3 steht,
das dadurch gekennzeichnet ist, dass
Verbindungen der Formel (II), 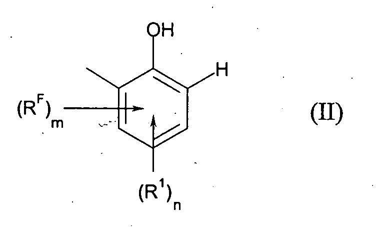 in der
R¹ und R^{F}, sowie n und m die vorstehend genannte Bedeutung besitzen,
- in Gegenwart von Urotropin und
- in Gegenwart von Säure
zu Verbindungen der Formel (I) umgesetzt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Alkyl beziehungsweise Alkylen beziehungsweise Alkoxy beziehungsweise Alkenyl bedeutet jeweils unabhängig einen geradkettigen, cyclischen, verzweigten oder unverzweigten Alkyl- beziehungsweise Alkylen- beziehungsweise Alkoxy- beziehungsweise Alkenyl-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, n-Nonyl, n-Decyl und n-Dodecyl.

C₁-C₈-Alkoxy steht beispielsweise für Methoxy, Ethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy.

Fluoralkyl steht jeweils unabhängig einen geradkettigen, cyclischen, verzweigten oder unverzweigten Alkylrest, der durch mindestens ein Fluoratom und gegebenenfalls weiter durch Chloratome und/oder Bromatome substituiert ist.

Beispielsweise steht C₁-C₁₂-Polyfluoralkyl für Trifluormethyl, Chlorfluormethyl, Difluormethyl, Difluorchlormethyl, 1,1,2,2-Tetrafluor-1-ethyl, 2-Chlor-2,1,1-trifluor-1-ethyl, 2,2,2-Trifluorethyl, Pentafluorethyl, 1,1-Dichlor-2,2,2-trifluorethyl, Heptafluorisopropyl, n-Nonafluorbutyl, Perfluorcyclopentyl, Perfluorcyclohexyl und Perfluordodecyl.

Im Folgenden werden die bevorzugten Substitutionsmuster für Verbindungen der Formeln (I) und (II) definiert:
- R¹: steht bevorzugt jeweils unabhängig für C₁-C₄-Alkyl oder Chlor, besonders bevorzugt für Methyl.
- n: steht bevorzugt für 0 oder 1, besonders bevorzugt für 0.
- R^{F}: steht bevorzugt für Fluor, C₁-C₄-Fluoralkyl, -O(C₁-C₄-Fluoralkyl) oder -S(C₁-C₄-Fluoralkyl), besonders bevorzugt für Trifluormethyl, Trifluormethylthio, Trifluormethoxy, Chlorfluormethyl, Chlorfluormethylthio, Chlorfluormethoxy, Difluormethoxy, Difluormethyl, Difluormethylthio, Difluormethoxy, Difluorchlormethyl, Difluorchlormethylthio, Difluorchlormethoxy, 1,1,2,2-Tetrafluor-1-ethyl, 1,1,2,2-Tetrafluor-1-ethylthio, 1,1,2,2-Tetrafluor-1-ethoxy, 2-Chlor-2,1,1-trifluor-1-ethyl, 2-Chlor-2,1,1-trifluor-1-ethylthio, 2-Chlor-2,1,1-trifluor-1-ethoxy, 2,2,2-Trifluorethyl, 2,2,2-Trifluorethylthio, 2,2,2-Trifluorethoxy, Pentafluorethyl, Pentafluorethylthio, Pentafluorethoxy, 1,1-Dichlor-2,2,2-trifluorethyl, 1,1-Dichlor-2,2,2-trifluorethylthio, 1,1-Dichlor-2,2,2-trifluorethoxy, Heptafluorisopropyl, n-Nonafluorbutyl, Perfluorcyclopentyl, Perfluorcyclohexyl und Perfluordodecyl.
- m: steht bevorzugt für den Fall, dass alle R^{F} für Fluor stehen für eine ganze Zahl von 1 bis 3, ansonsten für eins oder zwei, bevorzugt für eins.

Die Verbindungen der Formel (II) werden in Gegenwart von Urotropin und in Gegenwart von Säure in Verbindungen der Formel (I) überführt.

Das molare Verhältnis von Urotropin zu Verbindungen der Formel (II) kann beispielsweise 1:1 bis 10:1 betragen, bevorzugt 1:1 bis 5:1 und besonders bevorzugt 1,5:1 bis 2,5:1.

Das molare Verhältnis von Säure zu Verbindungen der Formel (II) kann beispielsweise 1:1 bis 100:1 betragen, bevorzugt 3:1 bis 10:1.

Als Säure werden bevorzugt solche eingesetzt, die bezogen auf ein wässriges Bezugssystem und 25°C einen pKs-Wert von 3 oder weniger aufweisen.

Besonders bevorzugte Säuren sind Perfluoralkylcarbonsäuren, ortho-Phosphorsäure und Polyphosphorsäuren, organische Sulfonsäuren, Chlor-, Brom- oder Iodwasserstoffsäure gegebenenfalls gelöst in Essigsäure, Hydrogensulfate und Schwefelsäure, wobei Bromwasserstoffsäure in Essigsäure, Polyphosphorsäuren, Methansulfonsäure und Trifluoressigsäure weiter und Trifluoressigsäure noch weiter bevorzugt ist.

Gegebenenfalls können der Reaktionsmischung auch organische Lösungsmittel zugesetzt werden, sofern sie im Wesentlichen unter den genannten Reaktionsbedingungen inert sind.

Vorzugsweise wird die Reaktion in der eingesetzten Säure durchgeführt.

Die Reaktionstemperatur kann beispielsweise 0°C bis 150°C betragen, bevorzugt 30 bis 150°C und besonders bevorzugt 70 bis 110°C.

Der Reaktionsdruck kann beispielsweise 0,5 bis 100 bar betragen, Umgebungsdruck ist bevorzugt.

Zur Umsetzung der Verbindungen der Formel (II) geht man beispielsweise so vor, dass die Verbindungen der Formel (II) und die Säure vorgelegt werden und anschließend die Zugabe von Urotropin erfolgt.

Die Aufarbeitung der Verbindungen der Formel (I) kann in an sich bekannter Weise durch Extraktion und anschließende Destillation oder bei 30 °C festen Verbindungen der Formel (I) durch Umkristallisation erfolgen.

Die Verbindungen der Formel (I) sind von der Erfindung ebenfalls umfasst. Für die Vorzugsbereiche gelten die vorstehend gemachten Angaben analog.

Als besonders bevorzugte Einzelverbindungen der Formel (I) seien genannt:
5-Fluor-2-hydroxy-3-methylbenzaldehyd, 5,6-Difluor-2-hydroxy-3-methylbenzaldehyd und 2-Hydroxy-3-methyl-5-(trifluormethoxy)benzaldehyd.

Vorzugsweise stellt man beispielsweise Verbindungen der Formel (II) dadurch her, dass Verbindungen der Formel (III), 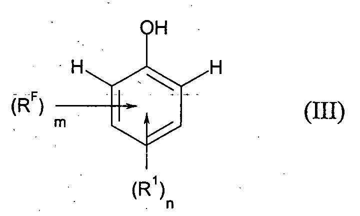 in der
- R¹, R^{F}: und m die vorstehend genannte Bedeutung und Vorzugsbereiche besitzen und
- n: für eine ganze Zahl zwischen 0 und 3-m steht,

a)
   - in Gegenwart von Formaldehyd und
   - in Gegenwart von sekundären Aminen der Formel (IV),

      HNR³R⁴ (IV)
   in der
   - R³ und R⁴: jeweils unabhängig voneinander für C₁-C₈-Alkyl stehen oder NR³R⁴ als Ganzes für einen cyclischen Aminorest mit insgesamt 4 bis 12 Kohlenstoffatomen steht,
   in Verbindungen der Formel (V) überführt, 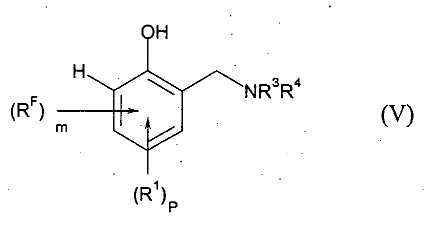 in der
   - R¹, R³, R⁴ und R^{F},: sowie n und m die vorstehend genannte Bedeutung und Vorzugsbereiche besitzen, und
b)
   die Verbindungen der Formel (V) zu Verbindungen der Formel (II) reduziert.

Die Verbindungen der Formel (II) sind als wichtige Intermediate von der Erfindung ebenfalls umfasst, wobei jedoch 2-Methyl-4-fluor-phenol ausgenommen ist. Für die Vorzugsbereiche gelten die vorstehend gemachten Angaben analog.

Weiterhin sind auch die Verbindungen der Formel (V) als wichtige Intermediate von der Erfindung umfasst, wobei jedoch 5-Fluor-2-hydroxy-N,N-dimethylbenzylamin ausgenommen ist. Für die Vorzugsbereiche gelten die vorstehend gemachten Angaben analog.

Gemäß Schritt a) werden die Verbindungen der Formel (III) in Gegenwart von Formaldehyd und in Gegenwart von sekundären Aminen der Formel (IV) in Verbindungen der Formel (V) überführt.

Das molare Verhältnis von Formaldehyd zu Verbindungen der Formel (III) kann beispielsweise 0,8 bis 10 betragen, bevorzugt 1,0 bis 10 und besonders bevorzugt 1,2 bis 3,6.

Das molare Verhältnis von sekundären Aminen der Formel (IV) zu Verbindungen der Formel (III) kann beispielsweise 0,8 bis 10 betragen, bevorzugt 1,0 bis 10 und besonders bevorzugt 1,05 bis 3,15.

Formaldehyd kann dabei beispielsweise als Paraformaldehyd und/oder in Form einer wässrigen Lösung eingesetzt werden, vorzugsweise in Form einer 32 bis 40 gew.-%igen Lösung.

Die sekundären Amine der Formel (IV) können beispielsweise in Substanz oder, sofern möglich, in Form wässriger Lösungen eingesetzt werden. Besonders bevorzugt wird Dimethylamin in Form einer wässrigen Lösung eingesetzt.

Die Reaktionstemperatur kann beispielsweise -40°C bis 120°C betragen, bevorzugt -10 bis 40°C und besonders bevorzugt -5 bis 10°C.

Der Reaktionsdruck kann beispielsweise 0,5 bis 100 bar betragen, Umgebungsdruck ist bevorzugt.

Die Reaktionsdauer kann 10 min bis 72 Stunden betragen, bevorzugt sind 3 Stunden bis 24 Stunden.

Zur Umsetzung geht man beispielsweise so vor, dass die Verbindungen der Formel (III) und die sekundären Amine der Formel (IV) vorgelegt werden und anschließend die Zugabe des Formaldehyds erfolgt.

Die Aufarbeitung der Verbindungen der Formel (V) kann in an sich bekannter Weise durch Extraktion und anschließende Destillation oder bei 30 °C festen Verbindungen der Formel (V) durch Umkristallisation erfolgen.

Die Reduktion gemäß Schritt b) kann dabei vorteilhafterweise in Gegenwart von Wasserstoff und Hydrierkatalysator durchgeführt werden.

Bevorzugte Hydrierkatalysatoren sind beispielsweise Metalle oder Metallverbindungen wie Salze oder Komplexe von Nickel, Palladium, Platin, Cobalt, Rhodium, Iridium und Ruthenium, wobei Metalle wie Nickel oder Palladium bevorzugt sind. Vorzugsweise werden Metalle in fein verteilter Form wie zum Beispiel als Raney-Metall oder auf ein Trägermaterial aufgebracht verwendet.

Besonders bevorzugt wird die Reduktion mit Wasserstoff und Raney-Nickel und/oder Palladium auf Kohle verwendet.

Die Reduktion kann beispielsweise bei einer Reaktionstemperatur von 20°C bis 200°C, bevorzugt 50 bis 180°C und besonders bevorzugt 80 bis 150°C durchgeführt werden.

Der Wasserstoffpartialdruck kann bei der Reduktion beispielsweise 0,1 bis 180 bar betragen, bevorzugt 10 bis 150 bar und besonders bevorzugt 40 bis 120 bar.

Gegebenenfalls und vorzugsweise kann die Reduktion in Gegenwart von Lösungsmittel durchgeführt werden, sofern sie im Wesentlichen unter den gewählten Reduktionsbedingungen inert sind.

Geeignete Lösungsmittel sind beispielsweise aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Alkohole wie beispielsweise Methanol, Ethanol und iso-Propanol, Carbonsäuren wie beispielsweise Essigsäure oder Mischungen von Lösungsmitteln.

Die Reaktionsdauer bei der Reduktion kann 10 min bis 200 Stunden betragen, bevorzugt sind 5 bis 100 Stunden.

In einer besonders bevorzugten Ausführungsform wird die Reduktion in Gegenwart von Palladium auf Aktivkohle und in Gegenwart von Essigsäure bei einem Wasserstoffpartialdruck von 40 bis 120 bar ausgeführt.

Die erfindungsgemäß erhältlichen Verbindungen der Formeln (I) und (II) eignen sich insbesondere in einem Verfahren zur Herstellung von Wirkstoffen wie beispielsweise von Wirkstoffen für Arzneimitteln. Bevorzugte Wirkstoffe für Arzneimittel sind solche, die zur Behandlung von Herz-Kreislaufmitteln eingesetzt werden. Insbesondere sind das diejenigen die in der WO-A/19780 beschrieben sind.

Ein wesentlicher Vorteil der Erfindung ist, dass die Verbindungen der Formel (I) und (II) in einfacher Weise aus gut verfügbaren Edukten hergestellt werden können. Weiterhin stellen die erfindungsgemäßen Verbindungen der Formeln (I) und (II) wertvolle Ausgangsprodukte für die Herstellung von Wirkstoffen insbesondere für Arzneimittel dar.

### Beispiele

### Beispiel 1

### Herstellung von 5-Fluor-2-hydroxy-3-methylbenzaldehyd

84 g 4-Fluor-2-methylphenol werden in 512 ml Trifluoressigsäure gelöst und portionsweise mit 181 g Hexamethylentetramin versetzt. Nach beendeter Zugabe wird die Reaktionslösung für 5 Stunden auf 100°C erhitzt. Nach dem Abkühlen werden zuerst 80 ml 50%ige Schwefelsäure und anschließend 480 ml Wasser zugetropft, dann lässt man die Lösung 3 Stunden weiter bei Raumtemperatur rühren. Die Reaktionslösung wird dreimal mit Dichlormethan extrahiert. Die vereinigten Extrakte werden einmal mit Wasser gewaschen, getrocknet und das Lösungsmittel wird im Vakuum abdestilliert. Der Rückstand wird mehrfach mit n-Hexan extrahiert, die Extrakte vereinigt und das Lösungsmittel im Vakuum abdestilliert. Das Rohprodukt wird anschließend fraktioniert destilliert. Man erhält 25 g (25% der Theorie) eines hellgelben Feststoffs mit einem Schmelzpunkt von 49-52°C und einem Siedepunkt von 70-72°C bei 7 mbar.

Das ¹H-NMR-Spektrum enthielt folgende charakteristischen Absorptionen (CDCl₃, δ/ppm): 1.08 (s, 1 H, OH); 9.83 (s, 1 H, CHO); 7.14, 7.07 (2 x dd, 2 H, H-4, H-6); 2.28 (s, 3 H, CH₃).

### Beispiel 2

### Herstellung von 5,6-Difluor-2-hydroxy-3-methylbenzaldehyd

30 g 4,5-Difluor-2-methylphenol werden in 180 ml Trifluoressigsäure gelöst und portionsweise mit 57,2 g Hexamethylentetramin versetzt. Nach beendeter Zugabe wird die Reaktionslösung für4 Stunden auf 97-100°C erhitzt. Nach dem Abkühlen werden zuerst 60 ml 50%ige Schwefelsäure und anschließend 300ml Wasser zugetropft, dann lässt man die Lösung 2 Stunden weiter bei Raumtemperatur rühren. Die Reaktionslösung wird dreimal mit Methyl-tert.Butylether extrahiert. Die vereinigten Extrakte werden einmal mit Wasser gewaschen, getrocknet und das Lösungsmittel im Vakuum abdestilliert. Der Rückstand wird mehrfach mit n-Hexan extrahiert, die Extrakte vereinigt und das Lösungsmittel im Vakuum abdestilliert. Man erhält 12,5 g (33% der Theorie) eines hellgelben Feststoffs mit einem Schmelzpunkt von 53-56°C.

Das ¹H-NMR-Spektrum enthielt folgende charakteristischen Absorptionen (CDCl₃, δ/ppm): 11.39 (bs, 1 H, OH); 10.24 (s, 1 H, CHO); 7.23 (m, 1 H, H-4); 2.22 (s, 3 H, CH₃).
Mittels GC-MS erhält man folgendes Spektrum (EI, 70eV, I/%): 172 (100, M⁺).

### Beispiel 3

### Herstellung von 2-Hydroxy-3-methyl-5-(trifluormethoxy)benzaldehyd

100 g 2-Methyl-4-(trifluormethoxy)phenol werden in 600ml Trifluoressigsäure gelöst und portionsweise mit 144 g Hexamethylentetramin versetzt. Nach beendeter Zugabe wird die Reaktionslösung für 16 Stunden auf 100°C erhitzt. Nach dem Abkühlen werden zuerst 200 ml 50%ige Schwefelsäure, danach 700 ml Wasser zugetropft, dann lässt man die Lösung 3 Stunden weiter bei Raumtemperatur rühren. Es wird dreimal mit Methyl-tert.-butylether extrahiert und die vereinigten organischen Phasen werden einmal mit Wasser gewaschen, getrocknet und das Lösungsmittel im Vakuum abdestilliert. Der Rückstand wird mehrfach mit n-Hexan extrahiert und von den vereinigten Extrakten wird das Lösungsmittel im Vakuum abdestilliert. Das Produkt kann durch Destillation weiter gereinigt werden. Man erhält 40 g (34% der Theorie) einer schwachgelben Flüssigkeit mit einem Siedepunkt von 65-67°C bei 4 mbar.

Das ¹H-NMR-Spektrum enthielt folgende charakteristischen Absorptionen (DMSO-d6, δ/ppm): 11.03 (bs, 1 H, OH); 10.12 (s, 1 H, CHO); 7.59, 7.48 (2 x m, 2 H, H-4,H-6); 2.26 (s, 3 H, CH₃).
Mittels GC-MS erhält man folgendes Spektrum (EI, 70eV, I/%): 220 (100, M⁺).

### Beispiel 4

### Herstellung von 4,5-Difluor-2-hydroxy-N,N-dimethylbenzylamin

400 g 3,4-Difluorphenol werden in 408 ml 40%iger wässriger Dimethylamin-Lösung vorgelegt und auf 0°C gekühlt. Bei 0-5°C werden 276 ml 37%ige wässrige Formaldehyd-Lösung innerhalb von 60 min. zugetropft. Es wird noch 2 Stunden bei 5-10°C gehalten und anschließend 20 Std. bei Raumtemperatur gerührt. Der Ansatz wird mit 600 ml Wasser versetzt. Man trennt die organische Phase ab, extrahiert die wässrige Phase zweimal mit Dichlormethan, trocknet die vereinigten organischen Phasen und destilliert das Lösungsmittel im Vakuum ab. Das Rohprodukt wird anschließend im Vakuum fraktioniert destilliert. Man erhält 395 g (65% der Theorie) einer farblosen Flüssigkeit mit einem Siedepunkt von 93°C bei 16 mbar.

Das ¹H-NMR-Spektrum enthielt folgende charakteristischen Absorptionen (CDCl₃, δ/ppm): 11.32 (s, 1 H, OH); 7.77, 7.60 (2 m, 2 H, H-3, H-6); 3.57 (s, 2 H, CH₂); 2.32 (s, 6 H, N(CH₃)₂).
Mittels GC-MS erhält man folgendes Spektrum (EI, 70eV, I/%): 187 (100, M⁺); 143 (36, (M-N(CH₃)₂)⁺).

### Beispiel 5

### Herstellung von 2-Hydroxy-5-(trifluormethoxy)-N,N-dimethylbenzylamin

130 g 4-Trifluormethoxyphenol werden in 97 ml 40%iger wässriger Dimethylamin-Lösung vorgelegt und auf 3°C gekühlt. Bei 0-5°C werden 66 ml 37%ige wässrige Formaldehyd-Lösung innerhalb von 45min. zugetropft. Es wird noch 2 Stunden bei 5-10°C gehalten und anschließend 19 Std. bei Raumtemperatur gerührt. Es wird erneut auf 0°C abgekühlt, 4,9 ml 40%ige wässrige Dimethylamin-Lösung zugesetzt und anschließend 3,3 ml 37%ige wässrige Formaldehyd-Lösung zugetropft. Man lässt 3 Stunden bei Raumtemperatur nachrühren. Der Ansatz wird mit 100 ml Wasser versetzt. Man trennt die organische Phase ab, extrahiert die wässrige Phase zweimal mit Dichlormethan, trocknet die vereinigten organischen Phasen und destilliert das Lösungsmittel im Vakuum ab. Das Rohprodukt wird anschließend im Vakuum fraktioniert destilliert. Man erhält 118 g (69 % der Theorie) einer hellgelben Flüssigkeit mit einem Siedepunkt von 110-112°C bei 18 mbar.

Das ¹H-NMR-Spektrum enthielt folgende charakteristischen Absorptionen (CDCl₃, δ/ppm): 11.08 (bs, 1 H, OH); 7.03 (dd, 1 H, J _{H4-H3}= 8.9 Hz, J _{H-4-H6}= 2.4 Hz, H-4); 6.84 (d, 1H, J_{H-6, H-4}= 2.3 Hz, H-6); 6.80 (d, 1 H, J_{H3-H4} = 8.8 Hz, H-3); 3.62 (s, 2 H, CH₂); 2.32 (s, 3 H, CH₃).
Mittels GC-MS erhält man folgendes Spektrum (EI, 70eV, I/%): 235 (100, M⁺); 191 (19, (M-N(CH₃)₂)⁺).

### Beispiel 6

### Herstellung von 6-Hydroxy-2,3,4-trifluor-N,N-dimethylbenzylamin

400 g 3,4,5-Trifluorphenol werden in 359 ml 40%iger wässriger Dimethylamin-Lösung vorgelegt und auf 0°C gekühlt. Bei 0-5°C werden 243 ml 37%ige wässrige Formaldehyd-Lösung innerhalb von 90min. zugetropft. Anschließend wird 20 Std. bei Raumtemperatur gerührt. Der Ansatz wird mit 600 ml Wasser und 500 ml Dichlormethan versetzt. Man trennt die organische Phase ab, extrahiert die wässrige Phase einmal mit Dichlormethan, trocknet die vereinigten organischen Phasen und destilliert das Lösungsmittel im Vakuum ab. Das Rohprodukt wird in 500ml Wasser aufgenommen und unter Kühlung im Eisbad mit verdünnter Salzsäure auf pH 1-2 eingestellt. Die saure Lösung wird einmal mit Dichlormethan extrahiert und anschließend unter Kühlung im Eisbad mit verdünnter Natronlauge auf pH 8-9 gestellt. Die alkalische Reaktionslösung wird dreimal mit Dichlormethan extrahiert, die vereinigten organischen Phasen werden einmal mit Wasser gewaschen, getrocknet und das Lösungsmittel im Vakuum abdestilliert. Das Rohprodukt wird anschließend im Vakuum fraktioniert destilliert. Man erhält 305 g (55% der Theorie) einer hellgelben Flüssigkeit mit einem Siedepunkt von 96°C bei 10 mbar.

Das ¹H-NMR-Spektrum enthielt folgende charakteristischen Absorptionen (CDCl₃, δ/ppm): 11.70 (s, 1 H, OH); 6.41 (m, 1 H, H-5); 3.71 (s, 2 H, CH₂); 2.37 (s, 6 H, N(CH₃)₂).

### Beispiel 7

### Herstellung von 2-Hydroxy-4-(trifluormethyl)-N,N-dimethylbenzylamin

195 g 3-Trifluormethylphenol werden in 160 ml 40%iger wässriger Dimethylamin-Lösung vorgelegt und auf 15°C gekühlt. Bei 155°C werden 108 ml 37%ige wässrige Formaldehyd-Lösung innerhalb von 40 min. zugetropft. Anschließend wird 20 Std. bei Raumtemperatur gerührt. Man kühlt erneut auf 15°C ab, versetzt mit 8 ml 40%iger wässriger Dimethylamin-Lösung und tropft 5,5 ml 37%ige wässrige Formaldehyd-Lösung zu. Anschließend wird 4,5 Stunden bei Raumtemperatur nachgerührt. Man kühlt erneut auf 15°C ab, versetzt mit 32 ml 40%iger wässriger Dimethylamin-Lösung und tropft 21 ml 37%ige wässrige Formaldehyd-Lösung zu. Anschließend wird 17 Stunden bei Raumtemperatur nachgerührt. Der Ansatz wird mit 150 ml Wasser versetzt. Man trennt die organische Phase ab, extrahiert die wässrige Phase zweimal mit Dichlormethan, wäscht die vereinigten organischen Phasen einmal mit Wasser und destilliert das Lösungsmittel im Vakuum ab. Das Rohprodukt wird in 250ml Wasser aufgenommen und unter Kühlung im Eisbad mit verdünnter Salzsäure auf pH 1-2 eingestellt. Die saure Lösung wird einmal mit Dichlormethan extrahiert und anschließend unter Kühlung im Eisbad mit verdünnter Natronlauge auf pH 11 gestellt. Die alkalische Reaktionslösung wird zweimal mit Dichlormethan extrahiert, die vereinigten organischen Phasen werden einmal mit Wasser gewaschen, getrocknet und das Lösungsmittel im Vakuum abdestilliert. Das Rohprodukt wird anschließend im Vakuum fraktioniert destilliert. Man erhält 186 g (71% der Theorie) einer hellgelben Flüssigkeit mit einem Siedepunkt von 91°C bei 14 mbar.

Das ¹H-NMR-Spektrum enthielt folgende charakteristischen Absorptionen (CDCl₃, δ/ppm): 7.08-6.98 (m, 3 H, H-3, H-5, H-6); 3.67 (s, 2 H, CH₂); 2.32 (s, 6 H, N(CH₃)₂).

### Beispiel 8

### Herstellung von 4,5-Difluor-2-methylphenol

214 g 4,5-Difluor-2-hydroxy-N,N-dimethylbenzylamin werden in 700ml Essigsäure in einem Autoklaven vorgelegt, mit 50g Palladium/Aktivkohle (5 %ig) versetzt und mit 70 bar Wasserstoff für 26 Stunden auf 100°C erhitzt. Der Autoklav wird abgekühlt und entspannt. Man filtriert den Katalysator ab, spült ihn mit Essigsäure und versetzt das Filtrat mit 3000 ml Wasser. Diese wässrige Lösung wird dreimal mit Methyl-tert.Butylether extrahiert. Die vereinigten organischen Phasen werden einmal mit Wasser gewaschen, getrocknet, und das Lösungsmittel wird im Vakuum abdestilliert. Das Rohprodukt wird im Vakuum fraktioniert destilliert. Das Destillat kann durch Kristallisation weiter gereinigt werden. Man erhält 98 g (58% der Theorie) eines farblosen Feststoffs mit einem Schmelzpunkt von 68-70°C und einem Siedepunkt von 78-80°C bei 13 mbar.

Das ¹H-NMR-Spektrum enthielt folgende charakteristischen Absorptionen (CDCl₃, δ/ppm): 6.92, 6.62 (2 m, 2 H, H-3, H-6); 4.82 (s, 1 H, OH); 2.19 (2, 3 H, CH₃). Mittels GC-MS erhält man folgendes Spektrum (EI, 70eV, I/%): 144 (100, M⁺); 126 (21, (M-F+H)⁺)

## Patentansprüche

1. Verfahren zur Herstellung von Verbindungen der Formel (I), 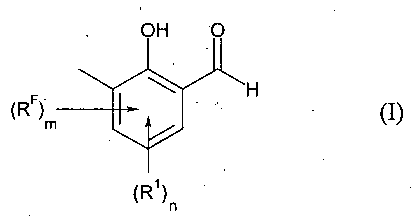 in der
R¹ jeweils unabhängig für C₁-C₁₂-Alkyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 3-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht,
**dadurch gekennzeichnet, dass**
Verbindungen der Formel (II), 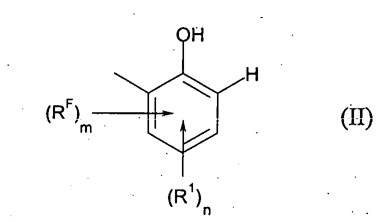 in der R¹ und R^{F}, sowie n und m die vorstehend genannte Bedeutung besitzen,
• in Gegenwart von Urotropin und
• in Gegenwart von Säure
zu Verbindungen der Formel (I) umgesetzt werden.

2. Verfahren nach Anspruch 1, **dadurch gekennzeichnet, dass** R¹ jeweils unabhängig für C₁-C₄-Alkyl oder Chlor steht.

3. Verfahren nach mindestens einem der Ansprüche 1 bis 2, **dadurch gekennzeichnet, dass** n für 0 oder 1 steht.

4. Verfahren nach mindestens einem der Ansprüche 1 bis 3, **dadurch gekennzeichnet, dass** R^{F} für Fluor, C₁-C₄-Fluoralkyl, -O(C₁-C₄-Fluoralkyl) oder -S(C₁-C₄-Fluoralkyl) steht.

5. Verfahren nach mindestens einem der Ansprüche 1 bis 4, **dadurch gekennzeichnet, dass** das molare Verhältnis von Urotropin zu Verbindungen der Formel (II) 1:1 bis 10:1 beträgt.

6. Verfahren nach mindestens einem der Ansprüche 1 bis 5, **dadurch gekennzeichnet, dass** das molare Verhältnis von Säure zu Verbindungen der Formel (II) 1:1 bis 100:1 beträgt.

7. Verfahren nach mindestens einem der Ansprüche 1 bis 6, **dadurch gekennzeichnet, dass** als Säure solche eingesetzt werden, die bezogen auf ein wässriges Bezugssystem und 25°C einen pKs-Wert von 3 oder weniger aufweisen.

8. Verfahren nach mindestens einem der Ansprüche 1 bis 7, **dadurch gekennzeichnet, dass** als Säuren Perfluoralkylcarbonsäuren, ortho-Phosphorsäure und Polyphosphorsäuren, organische Sulfonsäuren, Chlor-, Brom- oder Iodwasserstoffsäure gegebenenfalls gelöst in Essigsäure, Hydrogensulfate und Schwefelsäure eingesetzt werden.

9. Verfahren nach mindestens einem der Ansprüche 1 bis 8, **dadurch gekennzeichnet, dass** die Verbindungen der Formel (II) dadurch hergestellt werden, dass Verbindungen der Formel (III), 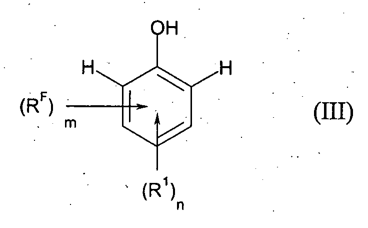 in der
R¹, R^{F} und m die vorstehend genannte Bedeutung und Vorzugsbereiche besitzen und
n für eine ganze Zahl zwischen 0 und 3-m steht,
a)
• in Gegenwart von Formaldehyd und
• in Gegenwart von sekundären Aminen der Formel (IV),
HNR³R⁴ (IV)
in der
R³ und R⁴ jeweils unabhängig voneinander für C₁-C₈-Alkyl stehen oder NR³R⁴ als Ganzes für einen cyclischen Aminorest mit insgesamt 4 bis 12 Kohlenstoffatomen steht,
in Verbindungen der Formel (V) überführt, 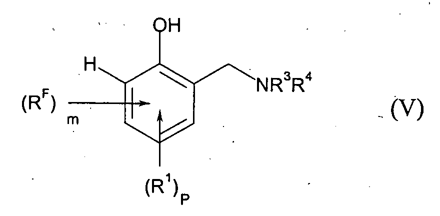 in der
R¹, R³, R⁴ und R^{F}, sowie n und m die vorstehend genannte Bedeutung und Vorzugsbereiche besitzen, und
b)
die Verbindungen der Formel (V) zu Verbindungen der Formel (II) reduziert.

10. Verfahren nach Anspruch 9, **dadurch gekennzeichnet, dass** im Schritt a) das molare Verhältnis von Formaldehyd zu Verbindungen der Formel (III) 0,8 bis 10 beträgt.

11. Verfahren nach mindestens einem der Ansprüche 9 bis 10, **dadurch gekennzeichnet, dass** im Schritt a) das molare Verhältnis von sekundären Aminen der Formel (IV) zu Verbindungen der Formel (III) 0,8 bis 10 beträgt.

12. Verfahren nach mindestens einem der Ansprüche 9 bis 11, **dadurch gekennzeichnet, dass** die Reduktion gemäß Schritt b) in Gegenwart von Wasserstoff und Hydrierkatalysator durchgeführt wird.

13. Verfahren nach Anspruch 12, **dadurch gekennzeichnet, dass** als Hydrierkatalysator Metalle oder Metallverbindungen wie Salze oder Komplexe von Nickel, Palladium, Platin, Cobalt, Rhodium, Iridium und Ruthenium eingesetzt werden.

14. Verfahren nach mindestens einem der Ansprüche 12 bis 13, **dadurch gekennzeichnet, dass** die Reduktion gemäß Schritt b) bei einer Reaktionstemperatur von 20°C bis 200°C und einem Wasserstoffpartialdruck von 0,1 bis 180 bar erfolgt.

15. Verbindungen der Formel (I), 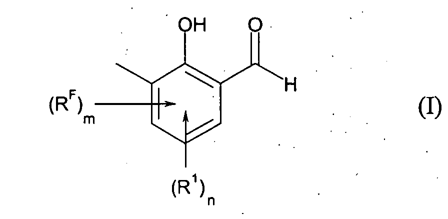 in der
R¹ jeweils unabhängig für C₁-C₁₂-Alkyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 3-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht.

16. 5-Fluor-2-hydroxy-3-methylbenzaldehyd, 5,6-Difluor-2-hydroxy-3-methylbenzaldehyd und 2-Hydroxy-3-methyl-5-(trifluormethoxy)benzaldehyd.

17. Verbindungen der Formel (II), 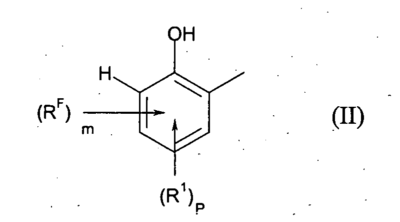 in der
R¹ jeweils unabhängig für C₁-C₁₂-Alkyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 3-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht.
wobei 4-Fluor-2-methylphenol ausgenommen ist.

18. Verbindungen der Formel (V), 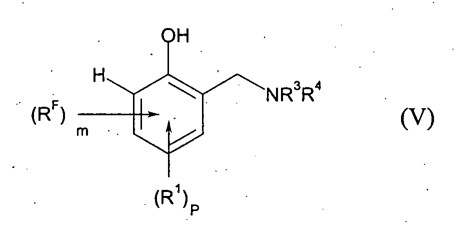 in der
R¹ jeweils unabhängig für C₁-C₁₂-Alkyl, Chlor oder Brom oder für Reste der Formeln (IIa) oder (IIb) steht,
A-B-D-E (IIa)
A-E (IIb)
in denen unabhängig voneinander
A fehlt oder für einen C₁-C₈-Alkylenrest steht und
B fehlt oder für Sauerstoff, Schwefel oder NR² steht,
wobei R² Wasserstoff oder C₁-C₈-Alkyl bedeutet und
D für eine Carbonyl-Gruppe steht und
E für C₁-C₈-Alkyl, C₁-C₈-Alkoxy, NH(C₁-C₈-Alkyl) oder N(C₁-C₈-Alkyl)₂ oder für einen cyclischen Aminorest mit 4 bis 12 Kohlenstoffatomen steht und
n für eine ganze Zahl von 0 bis 3-m steht und
R^{F} für Fluor, C₁-C₁₂-Fluoralkyl, -O(C₁-C₁₂-Fluoralkyl) oder -S(C₁-C₁₂-Fluoralkyl) steht und
m für eine ganze Zahl von 1 bis 3 steht und
R³ und R⁴ jeweils unabhängig voneinander für C₁-C₈-Alkyl stehen oder NR³R⁴ als Ganzes für einen cyclischen Aminorest mit insgesamt 4 bis 12 Kohlenstoffatomen steht,
wobei 5-Fluor-2-hydroxy-N,N-dimethylbenzylamin ausgenommen ist.

19. 4,5-Difluor-2-hydroxy-N,N-dimethylbenzylamin, 2-Hydroxy-5-(trifluormethoxy)-N,N-dimethylbenzylamin, 6-Hydroxy-2,3,4-trifluor-N,N-dimethylbenzylamin und 2-Hydroxy-4-(trifluormethyl)-N,N-dimethylbenzylamin.

20. Verwendung von Verbindungen gemäß mindestens einem der Ansprüche 15 bis 19 oder Verbindungen die gemäß einem Verfahren nach mindestens einem der Ansprüche 1 bis 14 hergestellt wurden zur Herstellung von Wirkstoffen für Arzneimitteln.

21. Verwendung nach Anspruch 20, **dadurch gekennzeichnet, dass** Wirkstoffe für Arzneimittel solche sind, die zur Behandlung von Herz-Kreislaufbeschwerden und -erkrankungen eingesetzt werden.
